**Europäisches Patentamt**

(19) **European Patent Office**

**Office européen des brevets**

(11) Publication number : **0 293 975 B1**

# EUROPEAN PATENT SPECIFICATION

(12)

(45) Date of publication of patent specification :
24.07.91 Bulletin 91/30

(51) Int. Cl.⁵ : **A61K 31/43, A61K 31/545, A61K 47/00**

(21) Application number : 88201049.9

(22) Date of filing : 25.05.88

(54) Dry, rapidly soluble, compositions of beta-lactam antibiotics.

(30) Priority : 27.05.87 EP 87201002

(43) Date of publication of application :
07.12.88 Bulletin 88/49

(45) Publication of the grant of the patent :
24.07.91 Bulletin 91/30

(84) Designated Contracting States :
AT BE CH DE ES FR GB GR IT LI NL SE

(56) References cited :
WO-A-86/03675
FR-A- 2 096 949
FR-A- 2 124 499
GB-A- 1 504 767
GB-A- 2 126 479
US-A- 3 459 858

(73) Proprietor : **AKZO N.V.**
**Velperweg 76**
**NL-6824 BM Arnhem (NL)**

(72) Inventor : **Tan, Hong Sheng**
**Buizerdstraat 94**
**NL-2665 TG Bleiswijk (NL)**
Inventor : **van Sinderen, Hendrik**
**Johan Vermeerstraat 35**
**NL-2162 BK Lisse (NL)**
Inventor : **Vork, Edoaldus**
**Corantijnstraat 16**
**NL-2071 VE Santpoort (NL)**

(74) Representative : **Hermans, Franciscus G.M. et al**
**Patent Department AKZO N.V. Pharma**
**Division P.O. Box 20**
**NL-5340 BH Oss (NL)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to pharmaceutical compositions of amphoteric β-lactam antibiotics, ready for dissolution in water.

More particularly, the invention relates to such compositions which are intended to be used for the medication of the drinking water of farm animals.

Although amphoteric β-lactam antibiotics such as amoxycillin, ampicillin and cephlexin are only sparingly soluble in water, the concentrations of β-lactam antibiotics which are required in the medication of the drinking water of farm animals are generally below the maximum solubility of these substances. For example, for amoxicillin trihydrate the maximum solubility according to the Merck Index, 10th Edition, is 4 g/l. However, the dissolution-rate of these compounds in practice appears to constitute a serious limiting factor for their use. When the relatively small amount of the antibiotic is simply poured into the relatively large volume of drinking water, as is the practice in the farm, a substantial portion of the antibiotic will require many hours to dissolve. In the meantime, not only the absorption of the antibiotic in drinking this water is adversely affected, but the incomplete solution also tends to clot and to block the installation, especially the drinking nipples.

It is therefore an aim of the present invention to provide pharmaceutical compositions of amphoteric β-lactam antibiotics which will have considerably accelerated dissolution rates, in the order of less than 10 minutes and preferably less than 5 minutes.

A dry pharmaceutical preparation, which may contain as its active substance amoxycillin, is described in the International patent application 86/03675. According to this application the active substance in a micronised powder form, is coated with a coherent layer of a small amount of a surfactant and a larger amount of a binder. The process of producing this preparation is relatively elaborate, involving preferably vacuum conditions.

It has now been found that the dissolution rate of amphoteric β-lactam antibiotics can be greatly improved by the simple addition (as distinct from a coherent coating) to said antibiotic of a relatively small amount of an excipient chosen from an alkali metal salt of an amino-acid carbonate. The amount of this excipient needed is in the range of 0.1-50% w/w, calculated from the total amount of the dry composition. The best results were obtained when sodium glycine carbonate was used, preferably in an amount of 0.5-30%, w/w, more preferably 0.5-20% w/w. A composition according to the invention may contain up to 99% of the amphoteric β-lactam antibiotic.

In French patent 2.305.194 sodium glycine carbonate is mentioned, but only as the source of carbon dioxide in an effervescent system, in conjunction with an acid such as citric acid.

In Japanese patent application 57.209.210 other amino acids including arginine, lysine, and their alkali metal salts, are described as pharmaceutical excipients. However, the document discloses only topical solutions, in which bis(2-pyridylthio-1-oxide)zinc (zinc pyrithione, an anti-dandruff compound) is the active compound, which has to be solubilised. According to this document, said excipients are used in an excess of 50-1000 times as much as the amount of zinc pyrithione.

The primary aim of the invention is already achieved by mixing the amphoteric β-lactam antibiotic with one of the excipients mentioned above. According to a further aspect of the invention, other pharmaceutically acceptable preparations such as granules may be made which may require the addition of a relatively small amount of a surfactant or a binder. It has been found that such excipients do not affect the dissolution rate.

The amount of the surfactant or binder is preferably between 0.01 and 20% w/w, and more preferably 0.5-10% w/w, calculated from the total amount of the dry composition.

Examples of suitable surfactants are sodium lauryl sulphate and sodium dioctyl sulphosuccinate, each in the amount of 0.01-2% w/w, a polyoxy ethylene alkyl ether, a polyoxyethylene alkyl acid ester and a polyoxyethylene sorbitan fatty acid ester, each in the amount of 0.1-20% w/w.

Examples of suitable binders are polyethylene glycol of mol. weight between 900-20000, preferably 1800-9000, more preferably 3000-9000, and polyvinyl pyrrolidone. Any type of polyvinyl pyrrolidone is suitable.

Other pharmaceutical excipients, such as lactose or saccharose, may also optionally be added although they do not affect the dissolution rate.

A very useful dry composition according to the invention comprises amoxycillin trihydrate, preferably in the amount of 70-99% w/w, sodium glycine carbonate, preferably in the amount of 5-20% w/w and more preferably 5-15%, and optionally polyethylene glycol 6000 (that is, of average mol. weight 5400-6000), preferably in the amount of 2-10% w/w.

The dry pharmaceutical composition according to the invention can be prepared by any of the convenient methods of dry blending generally used in the art. Preferably, this is followed by granulation, which again can be done with any of the known methods of dry or wet granulation.

The invention will now be illustrated by the following Examples.

Whenever powdered ingredients were used, they were first passed through a 1 mm sieve.

2

Example 1

9 g of amoxycillin trihydrate were thoroughly dry blended for 20 min. in a Turbula mixer, with 1 g of sodium glycine carbonate (mixture A), 1 g of polyethylene glycol 6000 (mixture B) and 0.5 g of sodium glycine carbonate plus 0.5 g of polyethylene glycol 6000 (mixture C).

The dissolution rate of the pure amoxycillin trihydrate and of the three mixtures was determined as follows.

A quantity of the powder, equivalent to 500 mg of the antibiotic, was added to 1 l of demineralized water at room temperature. Immediately thereafter the liquid was manually stirred by circularly moving a spatula for seven times. This stirring procedure was repeated once per minute for the first 10 minutes and further once per 10-15 minutes until the powder was dissolved or for a maximum of 120 min.

The results of this dissolution — rate test were as follows :

| Powder | Totally dissolved within | pH of the solution |
|---|---|---|
| Pure Amoxycillin trihydrate | > 120 min. | 4.30 |
| Mixture A | 6-7 min. | 6.50 |
| Mixture B | > 120 min. | 5.20 |
| Mixture C | 5-6 min. | 6.35 |

In the following Examples 2-9 granulates of the powder mixtures to be tested were used for each experiment.

Each time 10 g of the powder was mixed during 10 min in a 150 ml beaker with 3 ml of a granulation liquid (unless otherwise stated in the Examples, this was a 10% v/v solution of demineralized water in isopropanol). The wet mixture was dried overnight in an oven at 50°C. Finally the dried mass was passed through a 0.8 mm sieve.

When a binder and/or surfactant were used, they could be dissolved in the granulation liquid, when necessary by heating to about 50°C, before addition to the powder (Procedure 1). Alternatively, the binder and/or surfactant could be first mixed with the other powder ingredients before addition of the granulation liquid (Procedure 2).

The dissolution rate of the granulates, thus prepared, was estimated as described before in Example 1.

Example 2

| Granulate | A | B | C | D | E | F | G |
|---|---|---|---|---|---|---|---|
| Amoxycillin trihydrate | 10 g | 9 g | 9 g | 9 g | 9 g | 9 g | 9.5 g |
| Sodium glycine carbonate | -- | 1 g | -- | -- | 0.5 g | 0.5 g | -- |
| Polythylene-glycol 6000 | -- | -- | 1 g | 1 g | 0.5 g | 0.5 g | 0.5 g |
| Granulation procedure | | | 1 | 2 | 1 | 2 | 1 |
| Dissolution time (min.) | >120 | 3-4 | 120 | 120 | 2-3 | 2-3 | > 120 |
| pH of the solution | 4.7 | 7.0 | 5.3 | 5.3 | 6.4 | 6.2 | 5.1 |

This Example demonstrates that granulation of the mixture improves the dissolution rate further from about 6 min. to about 3 min. Both procedure 1 and 2 have the same effect.

Example 3

In this Example pure methylenechloride is used as the liquid for granulation and PVP instead of polyethyleneglycol is used as the binder.

| Granulate | A | B | C | D | E |
|---|---|---|---|---|---|
| Amoxycillin trihydrate | 9.5 g | 9.5 g | 9.0 g | 9.0 g | 9.5 g |
| Sodium glycine carbonate | -- | -- | 0.5 g | 0.5 g | -- |
| PVP K30 | -- | 0.5 g | -- | 0.5 g | 0.5 g |
| PVP K17 | 0.5 g | -- | 0.5 g | -- | -- |
| Granulation procedure | 2 | 2 | 2 | 1 | 1 |
| Dissolution time (min.) | $> 120$ | 25 | 3-4 | 3-4 | $> 120$ |
| pH of the solution | 5.4 | 5.4 | 6.3 | 6.4 | 5.3 |

Example 4

| Granulate | A | B | C | D | E | F | G | H |
|---|---|---|---|---|---|---|---|---|
| Ampicillin trihydrate | 9 g | 9 g | 9 g | 10 g | -- | -- | -- | -- |
| Cephalexin monohydrate | -- | -- | -- | -- | 9 g | 9 g | 9 g | 10 g |
| Sodium glycine carbonate | 0.5 g | -- | 1 g | -- | 0.5 g | -- | 1 g | -- |
| Polyethylene glycol 6000 | 0.5 g | 1 g | -- | -- | 0.5 g | 1 g | -- | -- |
| Granulation procedure | 1 | 1 | | | 1 | 1 | | |
| Dissolution time (min.) | 4-5 | >120 | 6-7 | >120 | 8-9 | 150 | 7-8 | >120 |
| pH of the solution | 6.2 | 4.6 | 6.5 | 4.1 | 6.4 | 5.2 | 6.4 | 5.4 |

6

Example 5

| Granulate | A | b | C | D | E | F | G |
|---|---|---|---|---|---|---|---|
| Amoxycillin trihydrate | 9.9 g | 9.9 g | 9.5 g | 9.5 g | 7.5 g | 5.0 g | 5.0 g |
| Sodium glycine carbonate | 0.1 g | 0.05 g | 0.5 g | 0.4 g | 2.0 g | 2.0 g | 2.0 g |
| Polythylene- glycol 6000 | -- | 0.05 g | -- | 0.1 g | -- | 0.5 g | -- |
| Lactose | -- | -- | -- | -- | 0.5 g | 2.5 g | 3.0 g |
| Granulation procedure | | 1 | | 1 | 2 | 1 | 2 |
| Dissolution time (min.) | 3-4 | 2-3 | 3-4 | 2-3 | 2-3 | 3-4 | 3-4 |
| pH of the solution | 5.7 | 5.6 | 6.5 | 6.5 | 7.5 | 7.7 | 7.7 |

Example 6

| Granulate | A | B | C | D |
|---|---|---|---|---|
| Amoxycillin trihydrate | 9 g | 9 g | 9 g | 9 g |
| Lactose | 0.5 g | 1 | -- | -- |
| Saccharose | -- | -- | 0.5 g | 1 |
| Polythylene glycol 6000 | 0.5 g | -- | 0.5 g | -- |
| Granulation procedure | 1 | 2 | 1 | 2 |
| Dissolution time (min.) | >120 | > 120 | > 120 | >120 |
| pH of the solution | 5.0 | 4.9 | 5.0 | 4.9 |

8

Example 7

| Granulate | A | B | C | D | E |
|---|---|---|---|---|---|
| Amoxycillin trihydrate | 9 g | 9 g | 9 g | 9 g | 9 g |
| Sodium glycine carbonate | 0.5 g | 0.5 g | -- | 0.5 g | -- |
| Polyethylene glycol 4000 | 0.5 g | -- | -- | -- | -- |
| Lactose | -- | 0.4 g | 0.9 g | 0.4 g | 0.9 g |
| Sodium dioctyl sulphosucci-nate | -- | 0.1 g | 0.1 g | -- | -- |
| Sodiumlauryl sulfate | -- | -- | -- | 0.1 g | 0.1 g |
| Granulation procedure | 1 | 1 | 1 | 1 | 1 |
| Dissolution time (min.) | 3-4 | 2-3 | > 120 | 2-3 | > 120 |
| pH of the solution | 6.8 | 6.5 | 5.3 | 6.5 | 5.1 |

# Example 8

| Granulate | A | B | C | D | E | F | G | H |
|---|---|---|---|---|---|---|---|---|
| Amoxycillin trihydrate | 9 g | 9 g | 9 g | 9 g | 9 g | 9 g | 9 g | 9 g |
| Sodiumglycine carbonate | 0.5 g | -- | 0.5 g | -- | 0.5 g | -- | 0.5 g | -- |
| Lactose | -- | 0.5 g | -- | 0.5 g | -- | 0.5 g | -- | 0.5 |
| Brij 35 (R)*) | 0.5 g | 0.5 g | -- | -- | -- | -- | -- | -- |
| Tween 20 (R)*) | -- | -- | 0.5 g | 0.5 g | -- | -- | -- | -- |
| Myrj 52 (R)*) | -- | -- | -- | -- | 0.5 g | 0.5 g | -- | -- |
| PVP K17 *) | -- | -- | -- | -- | -- | -- | 0.5 g | 0.5g |
| Granulation procedure | 1 | 1 | 1 | 1 | 1 | 1 | 1 | 1 |
| Dissolution time (min.) | 2-3 | 120 | 2-3 | 120 | 2-3 | 120 | 2-3 | 120 |
| pH of the solution | 7.0 | 5.1 | 6.5 | 5.1 | 6.5 | 5.1 | 6.5 | 5.2 |

*) Brij 35 is a pharmaceutical excipient of the polythylene Alkyl Ether type.
Tween 20 is an excipient of the Polyoxyethylene Sorbitan Fatty Acid Ester type
Myrj 52 is a Polyoxyethylene stearate.
PVP is Polyvinylpyrrolidone.

Example 9

| Granulate | A | B | C | D | E | F |
|---|---|---|---|---|---|---|
| Amoxycillin-trihydrate | 9 g | 9 g | 9 g | 9 g | 9 g | 9 g |
| L-Arginine | 0.5 g | 1 g | -- | -- | -- | -- |
| Glycine | -- | -- | 0.5 g | 1 g | -- | -- |
| L-Histidine | -- | -- | -- | -- | 1 g | -- |
| Sodium glutaminate | -- | -- | -- | -- | -- | 1 g |
| Polyethylene glycol 6000 | 0.5 g | -- | 0.5 g | -- | -- | -- |
| Granulation procedure | 1 | -- | 1 | -- | -- | -- |
| Dissolution time (min.) | 9-10 | 9-10 | > 120 | >120 | >120 | >120 |
| pH of the solution | 6.3 | 6.5 | 4.8 | 4.5 | 6.2 | 5.3 |

Example 10

675 g of amoxycillin trihydrate and 37.5 g of sodium glycine carbonate were mixed during 10 minutes in a Hobart planetary mixer.

Meanwhile a solution of 37.5 g of polyethylene glycol 6000 in 150 ml of a 1 : 9 (v/v) mixture of demineralized water and isopropanol was prepared in a beaker of 250 ml by heating up the mixture to ca. 55°C.

While mixing was continued, the solution was slowly added to the powder mixture. The beaker was rinsed with 75 ml of a 1 : 9 (v/v) mixture of demineralized water and isopropanol which subsequently was also added to the powder mixture. The mixing was continued during a further 20 minutes, and the wet mass transferred into a fluidised drier. The granulate was dried for about 1 hour with an inlet air temperature of 50°C. Finally the dried granulate was passed through a 0.8 mm sieve. It had a content (w/w) of 90% Amoxycillin trihydrate, 5% polyethlene glycol 6000 and 5% sodium glycine carbonate.

The dissolution time of 550 mg of this granulate into 1 l of demineralized water, determined as in Example 1, was 2-3 minutes. The pH of the solution was 6.6.

### Example 11

609 g of amoxycillin trihydrate and 103.5 g of sodium glycine carbonate were mixed for 10 minutes in a Hobart planetary mixer.

A solution of 37.5 g of polyethylene glycol 6000 in a mixture of 250 ml isopropanol and 20 ml of demineralized water was slowly added to the stirred powder mixture. Mixing was continued for another 20 minutes and the formed granulate transferred into a fluidised bed drier. The material was dried for about 30 minutes with an inlet air temperature of about 45°C, and then passed through a 0.8 mm sieve. It had a content (w/w) of 81,2% amoxycillin trihydrate, 13,8% sodium glycine carbonate and 5% polyethylene glycol 6000.

The dissolution time of 650 mg of this granulate, determined as in Example 1, was 2-3 minutes and the pH of the solution was 7.3.

### Example 12

731 g of amoxycillin trihydrate, 45 g of polyethylene glycol 6000 and 124 g of sodium glycine carbonate were mixed during 10 minutes in a Hobart planetary mixer. 345 ml of isopropanol was slowly added to the stirred powder mixture. The wet mass was stirred for another 20 minutes and then transferred into a fluidised bed drier. The granulate was dried for about 1 hour with an inlet air temperature of 45°C, and finally passed through a 0.8 mm sieve. It had a content (w/w) of 81.2% amoxycillin trihydrate, 13.8% sodium glycine carbonate and 5%· polyethylene glycol 6000. The dissolution time of 650 mg of the granulate, determined as in Example 1, was 2-3 minutes and the pH of the solution was 7.2.

### Claims

**Claims for the following Contracting States : AT, BE, CH/LI, DE, FR, IT, NL, SE, GB**

1. A pharmaceutical composition of an amphoteric β-lactam antibiotic, ready for dissolution in water, characterized in that it comprises an amount of 0.1% to 50% w/w of an alkali metal salt of a glycine carbonate.

2. A pharmaceutical composition according to claim 1, characterized in that the alkali metal salt of a glycine carbonate is sodium glycine carbonate.

3. A pharmaceutical composition according to claim 2, characterized in that the sodium glycine carbonate is present in an amount of 0.5% to 30% w/w.

4. A pharmaceutical composition according to any of claims 1-3, characterized in that it further comprises an amount of 0.01%-20% of a binder or surfactant.

5. A pharmaceutical composition according to claim 4, characterized in that it contains a binder, said binder being a polyethylene glycol of molecular weight between 900 and 20000, or polyvinylpyrrolidone.

6. A pharmaceutical composition according to claim 4, characterized in that a surfactant is present, and said surfactant is sodium lauryl sulphate in an amount of 0.01 to 2% w/w or sodium dioctyl sulphosuccinate in an amount of 0.01 to 2% w/w.

7. A pharmaceutical composition according to claim 4, characterized in that a surfactant is present, said surfactant being a polyoxyethylene alkyl ether, a polyoxyethylene fatty acid ester or a polyethylene sorbitan fatty acid ester, in an amount of 0.1 to 20% w/w.

8. A pharmaceutical composition according to claims 1-7, characterized in that it comprises 70 to 99% w/w of amoxycillin trihydrate, 0.5 to 20% w/w of sodium glycine carbonate, and, optionally 2 to 10% w/w of polyethylene glycol 6000 (average molecular weight 5400 to 6600).

9. A process for the preparation of a pharmaceutical composition ready for dissolution in water, of an amphoteric β-lactam antibiotic, characterized in that the amphoteric β-lactam antibiotic is blended with an amount of 0.1% to 50% w/w of an alkali metal salt of a glycine carbonate.

10. A process according to claim 9 characterized in that the blended powder is granulated by a method known per se.

11. A process according to claim 2 characterized in that a binder and/or a hydrophilic surfactant is added to the blended powder or to a granulation liquid before granulation.

**Claims for the following Contracting States : GR, ES·**

1. A process for the preparation of a pharmaceutical composition ready for dissolution in water, of an amphoteric β-lactam antibiotic, characterized in that the amphoteric β-lactam antibiotic is blended with an amount of 0.1% to 50% w/w of an alkalimetal salt of an glycine carbonate to from a blended powder.

2. A process according to claim 1 characterized in that the blended powder is granulated by a method known per se.

3. A process according to claim 2 characterized in that a binder is added to the blended powder or to a granulation liquid used therewith before granulation.

4. A process according to claim 2 characterized in that a hydrophilic surfactant is added to the blended powder or to the granulation liquid before granulation.

5. A process according to claim 2 characterized in that a binder and a hydrophilic surfactant are added to the blended powder or to the granulation liquid before granulation.

6. A process for the preparation of a pharmaceutical solution characterized in that the pharmaceutical preparation prepared according to claim 1 is dissolved into water.

**Patentansprüche**

**Patentansprüche für folgende Vertragsstaten : AT, BE, CH/LI, DE, FR, IT, NL, SE, GB**

1. Pharmazeutische Zusammensetzung eines amphoteren β-Lactam-Antibioticums, welche bereit ist zur Auflösung in Wasser, dadurch gekennzeichnet, dass sie eine Menge von 0,1 bis 50% (Gew./Gew.) eines Alkalimetallsalzes eines Glycincarbonates enthält.

2. Pharmazeutische Zusammensetzung nach Anspruch 1, dadurch gekennzeichnet, dass das Alkalimetallsalz eines Glycincarbonates Natriumglycincarbonat ist.

3. Pharmazeutische Zusammensetzung nach Anspruch 2, dadurch gekennzeichnet, dass das Natriumglycincarbonat in einer menge von 0,5 bis 30% (Gew./Gew.) vorhanden ist.

4. Pharmazeutische Zusammensetzung nach einem der Ansprüche 1 bis 3, dadurch gekennzeichnet, dass sie ausserdem eine Menge von 0,01 bis 20% eines Bindemittels oder eines oberflächenaktiven Mittels enthält.

5. Pharmazeutische Zusammensetzung nach Anspruch 4, dadurch gekennzeichnet, dass sie ein Bindemittel enthält, wobei dieses Bindemittel ein Polyäthylenglykol mit einem Molekulargewicht zwischen 900 und 20'000 oder Polyvinylpyrrolidon ist.

6. Pharmazeutische Zusammensetzung nach Anspruch 4, dadurch gekennzeichnet, dass ein oberflächenaktives Mittel zugegen ist und dieses oberflächenaktive Mittel Natriumlaurylsulfat in einer Menge von 0,01 bis 2% (Gew./Gew.) oder Natriumdioctylsulfosuccinat in einer Menge von 0,01 bis 2% (Gew./Gew.) ist.

7. Pharmazeutische Zusammensetzung nach Anspruch 4, dadurch gekennzeichnet, dass ein oberflächenaktives Mittel zugegen ist, wobei dieses oberflächenaktive Mittel ein Polyoxyäthylenalkyläther, ein Polyoxyäthylenfettsäureester oder ein Polyäthylensorbitanfettsäureester in einer Menge von 0,1 bis 20% (Gew./Gew.) ist.

8. Pharmazeutische Zusammensetzung nach den Ansprüchen 1 bis 7, dadurch gekennzeichnet, dass sie 70 bis 99% (Gew./Gew.) Amocyllin-trihydrat, 0,5 bis 20% (Gew./Gew.) Natriumglycincarbonat und gegebenenfalls 2 bis 10% (Gew./Gew.) Polyäthylenglykol 6000 (mittleres Molekulargewicht 5400 bis 6600) enthält.

9. Verfahren zur Herstellung einer pharmazeutischen Zusammensetzung eines amphoteren β-Lactam-Antibiotikums, welches zur Auflösung in Wasser bereit ist, dadurch gekennzeichnet, dass das amphotere β-Lactam-Antibiotikum mit einer Menge von 0,1 bis 50% (Gew./Gew.) eines Alkalimetallsalzes eines Glycincarbonates gemischt wird.

10. Verfahren nach Anspruch 9, dadurch gekennzeichnet, dass das vermischte Pulver nach einem an sich bekannten Verfahren granuliert wird.

11. Verfahren nach Anspruch 9, dadurch gekennzeichnet, dass ein Bindemittel und/oder ein hydrophiles oberflächenaktives Mittel zu dem vermischten Pulver oder zu einer Granulierflüssigkeit vor der Granulierung zugesetzt wird.

**Patentansprüche für folgende Vertragsstaaten : GR, ES**

1. Verfahren zur Herstellung eines pharmazeutischen Präparates, eines amphoteren β-Lactam-Antibiotikums, welches zur Auflösung in Wasser bereit ist, dadurch gekennzeichnet, dass das amphotere β-Lactam-Antibiotikum mit einer Menge von 0,1 bis 50% (Gew./Gew.) eines Alkalimetallsalzes eines Glycincarbonates gemischt wird.

2. Verfahren nach Anspruch 1, dadurch gekennzeichnet, dass das vermischte Pulver nach einem an sich bekannten Verfahren granuliert wird.

3. Verfahren nach Anspruch 2, dadurch gekennzeichnet, dass ein Bindemittel zu dem vermischten Pulver oder zu einer damit verwendeten Granulierflüssigkeit vor dem Granulieren zugesetzt wird.

4. Verfahren nach Anspruch 2, dadurch gekennzeichnet, dass ein hydrophiles oberflächenaktives Mittel zu dem vermischten Pulver oder zu der Granulierflüssigkeit vor dem Granulieren zugesetzt wird.

5. Verfahren nach Anspruch 2, dadurch gekennzeichnet, dass ein Bindemittel und ein hydrophiles oberflächenaktives Mittel zu dem vermischten Pulver oder zur Granulierflüssigkeit vor dem Granulieren zugesetzt werden.

6. Verfahren zur Herstellung einer pharmazeutischen Lösung, dadurch gekennzeichnet, dass das pharmazeutische Präparat hergestellt nach Anspruch 1 in Wasser aufgelöst wird.

## Revendications

**Revendications pour les Etats contractants suivants : AT, BE, CH/LI, DE, FR, IT, NL, SE, GB**

1. Composition pharmaceutique d'une bêtalactamine amphotère, prête à dissoudre dans l'eau, caractérisée par le fait qu'elle contient une quantité de 0,1% à 50% en poids d'un sel de métal alcalin d'un carbonate de glycine.

2. Composition pharmaceutique selon la revendication 1, caractérisée par le fait que le sel de métal alcalin d'un carbonate de glycine est le carbonate de glycine sodique.

3. Composition pharmaceutique selon la revendication 2, caractérisée par le fait que le carbonate de glycine sodique est présent en une quantité de 0,5% à 30% en poids.

4. Composition pharmaceutique selon l'une quelconque des revendications 1 à 3, caractérisée par le fait qu'elle contient de plus un liant ou un agent tensio-actif en une quantité de 0,01% à 20%.

5. Composition pharmaceutique selon la revendication 4, caractérisée par le fait qu'elle contient un liant, ledit liant étant un polyéthylène-glycol de poids moléculaire compris entre 900 et 20000, ou la polyvinylpyrrolidone.

6. Composition pharmaceutique selon la revendication 4, caractérisée par le fait qu'elle contient un agent tensio-actif, et ledit agent tensio-actif est le laurylsulfate de sodium en une quantité de 0,01 à 2% en poids ou le dioctylsulfosuccinate de sodium en une quantité de 0,01 à 2% en poids.

7. Composition pharmaceutique selon la revendication 4, caractérisée par le fait qu'elle contient un agent tensio-actif, ledit agent tensio-actif étant un éther alkylique de polyoxyéthylène, un ester d'acide gras de polyoxyéthylène ou un ester d'acide gras de polyéthylènesorbitanne, en une quantité de 0,1 à 20% en poids.

8. Composition pharmaceutique selon les revendications 1 à 7, caractérisée par le fait qu'elle contient 70 à 99% en poids de trihydrate d'amoxicilline, 0,5 à 20% en poids de carbonate de glycine sodique et, facultativement, 2 à 10% en poids de polyéthylène-glycol 6000 (poids moléculaire moyen de 5400 à 6600).

9. Procédé pour la préparation d'une composition pharmaceutique d'une bêtalactamine amphotère, prête à dissoudre dans l'eau, caractérisé par le fait que la bêtalactamine amphotère est mélangée avec une quantité de 0,1% à 50% en poids d'un sel de métal alcalin d'un carbonate de glycine.

10. Procédé selon la revendication 9, caractérisé par le fait que la poudre mélangée est granulée par une technique connue en soi.

11. Procédé selon la revendication 10, caractérisé par le fait qu'un liant et/ou un agent tensio-actif hydrophile sont ajoutés à la poudre mélangée ou à un liquide de granulation avant la granulation.

**Revendications pour les Etats contractants suivants : GR, ES**

1. Procédé pour la préparation d'une composition pharmaceutique d'une bêtalactamine amphotère, prête à dissoudre dans l'eau, caractérisé par le fait que la bêtalactamine amphotère est mélangée avec une quantité de 0,1% à 50% en poids d'un sel de métal alcalin d'un carbonate de glycine pour former une poudre mélangée.

2. Procédé selon la revendication 1, caractérisé par le fait que la poudre mélangée est granulée par une technique connue en soi.

3. Procédé selon la revendication 2, caractérisé par le fait qu'un liant est ajouté à la poudre mélangée ou à un liquide de granulation utilisé conjointement, avant la granulation.

4. Procédé selon la revendication 2, caractérisé par le fait qu'un agent tensio-actif hydrophile est ajouté à la poudre mélangée ou au liquide de granulation avant la granulation.

5. Procédé selon la revendication 2, caractérisé par le fait qu'un liant et un agent tensio-actif hydrophile sont ajoutés à la poudre mélangée ou au liquide de granulation avant la granulation.

6. Procédé pour la préparation d'une solution pharmaceutique, caractérisé par le fait que la préparation pharmaceutique, préparée selon la revendication 1, est dissoute dans l'eau.